# EUROPÄISCHE PATENTANMELDUNG

(11) **EP 3 335 741 A1**
(43) Veröffentlichungstag der Anmeldung: **20.06.2018**
(21) Anmeldenummer: 16204167.7
(22) Anmeldetag: 14.12.2016
(51) Int. Cl.: A61M 1/12

(54) **STEUERVORRICHTUNG FÜR EINE IMPLANTIERBARE HERZPUMPE MIT ZWEI IMPLANTIERBAREN STEUEREINHEITEN UND MIT EINEM MIT DIESEN VERBUNDENEN IMPLANTIERBAREN SCHALTER**

(71) Anmelder: Berlin Heart GmbH, 12247 Berlin (DE)
(72) Erfinder: FRISCHKE, Michael, 15834 Rangsdorf (DE); KALLENBACH, Sebastian, 34128 Kassel (DE)
(74) Vertreter: Pfenning, Meinig & Partner mbB

(57) **Zusammenfassung**

Die Anmeldung betrifft eine Steuervorrichtung (7) für eine implantierbare Herzpumpe (5). Die vorgeschlagene Steuervorrichtung umfasst eine implantierbare erste Steuereinheit, die in einem Hauptbetriebszustand zum Steuern von Betriebsparametern der Herzpumpe elektrisch mit der Herzpumpe verbunden ist. Weiterhin umfasst die Steuervorrichtung eine mit der ersten Steuereinheit elektrisch verbundene Schnittstelle (18) zur drahtlosen, transkutanen Datenübertragung und/oder zur drahtlosen, transkutanen Energieübertragung zwischen der ersten Steuereinheit und einer für eine extrakorporale Verwendung vorgesehenen weiteren Steuereinheit (3). Die Steuervorrichtung umfasst außerdem eine implantierbare zweite Steuereinheit, die in einem Hilfsbetriebszustand zum Steuern von Betriebsparametern der Herzpumpe elektrisch mit der Herzpumpe verbunden ist, und einen mit der ersten Steuereinheit und der zweiten Steuereinheit elektrisch verbundenen und implantierbaren Schalter (16, 17). Der Schalter ist eingerichtet, zwischen dem Hauptbetriebszustand und dem Hilfsbetriebszustand umzuschalten.

## Beschreibung

Die vorliegende Anmeldung liegt auf dem Gebiet der Medizintechnik und betrifft eine Steuervorrichtung für eine implantierbare Herzpumpe. Außerdem betrifft die vorliegende Anmeldung ein Verfahren zum Betrieb einer derartigen Steuervorrichtung.

Implantierbare Herzpumpen zur Unterstützung eines menschlichen Herzens sind aus dem Stand der Technik bekannt. Derartige Herzpumpen sind beispielsweise eingerichtet zum Fördern von Blut von einem Herzventrikel in die Aorta oder in die Pulmonalarterie und können beispielsweise als sog. LVAD (left ventricular assist device), RVAD (right ventricular assist device) oder BiVAD (bi-ventricular assist device) ausgeführt sein. In typischen Ausführungen sind eine elektrische Energieversorgung und eine Steuereinheit für die Herzpumpe vorgesehen, die außerhalb des Körpers angeordnet und über zumindest eine transkutane Leitung mit der Herzpumpe verbunden sind. Für den Fall, dass ein Fehler in der Steuereinheit auftritt, trägt ein Patient, bei dem die implantierbare Herzpumpe zum Einsatz kommt, typischerweise eine Ersatzsteuereinheit jederzeit mit sich. Üblicherweise ist die Steuereinheit eingerichtet, einen Alarm zu erzeugen, falls ein Fehler in dieser auftritt. Durch den Alarm wird der Patient aufgefordert, die Steuereinheit gegen die Ersatzsteuereinheit auszutauschen. Damit dieser einen Austausch der Steuereinheiten innerhalb kurzer Zeit durchführen kann, ist der Patient üblicherweise speziell geschult.

Bei den oben beschriebenen Systemen ist für die Energieversorgung und Steuerung der Herzpumpe eine Durchtrittsstelle durch die Haut notwendig, wodurch ein Infektionsrisiko des Patienten erhöht ist. Daher gibt es Bestrebungen, beispielsweise die Steuereinheit der Herzpumpe implantierbar auszuführen und die Herzpumpe und/oder die Steuereinheit drahtlos und transkutan mit Energie zu versorgen (sog. transkutaner Energietransfer, TET). Bei einer Verwendung eines derartigen Systems tritt allerdings der Nachteil auf, dass die implantierte Steuereinheit bei Auftreten eines Fehlers der Steuereinheit nicht ohne Weiteres durch den Patienten gegen eine Ersatzsteuereinheit austauschbar ist.

Es ist daher eine Aufgabe der vorliegenden Anmeldung, eine verbesserte Steuervorrichtung für eine implantierbare Herzpumpe vorzuschlagen, durch welche die Nachteile oben genannter Systeme überwunden werden. Insbesondere ist es eine Aufgabe der vorliegenden Anmeldung, eine Steuervorrichtung vorzuschlagen, die sich durch eine verbesserte Fehlersicherheit bei geringem Infektionsrisiko des Patienten auszeichnet.

Gelöst werden diese Aufgaben durch eine Steuervorrichtung mit den Merkmalen des Anspruchs 1. Vorteilhafte Weiterbildungen ergeben sich mit den Merkmalen der abhängigen Ansprüche und der Ausführungsbeispiele.

Die vorgeschlagene Steuervorrichtung für eine implantierbare Herzpumpe umfasst eine implantierbare erste Steuereinheit, die in einem Hauptbetriebszustand zum Steuern von Betriebsparametern der Herzpumpe elektrisch mit der Herzpumpe verbunden ist. Weiterhin umfasst die Steuervorrichtung eine mit der ersten Steuereinheit elektrisch verbundene Schnittstelle zur drahtlosen, transkutanen Datenübertragung und/oder zur drahtlosen, transkutanen Energieübertragung zwischen der ersten Steuereinheit und einer für eine extrakorporale Verwendung vorgesehenen weiteren Steuereinheit. Die Steuervorrichtung umfasst außerdem eine implantierbare zweite Steuereinheit, die in einem Hilfsbetriebszustand zum Steuern von Betriebsparametern der Herzpumpe elektrisch mit der Herzpumpe verbunden ist, und einen mit der ersten Steuereinheit und der zweiten Steuereinheit elektrisch verbundenen und implantierbaren Schalter. Der Schalter ist eingerichtet, zwischen dem Hauptbetriebszustand und dem Hilfsbetriebszustand umzuschalten. Die Betriebsparameter der Herzpumpe können beispielsweise eine Antriebsdrehzahl und/oder eine Rotordrehzahl und/oder einen Motorstrom umfassen oder daraus bestehen. Darüber hinaus können die Betriebsparameter auch Signale zur Ansteuerung von Sensoren sein. So kann es beispielsweise vorgesehen sein, dass die Sensoren im Hilfsbetriebszustand abgeschaltet werden oder beispielsweise bei geringerer Leistung betrieben werden.

Die Anmeldung betrifft des Weiteren eine Herzpumpeneinrichtung umfassend eine wie oben und/oder unten beschriebene Steuervorrichtung und eine mit dem Schalter elektrisch verbundene Herzpumpe. Des Weiteren betrifft die vorliegende Anmeldung ein System mit einer wie oben und/oder unten beschriebenen Steuervorrichtung oder einer wie oben und/oder unten beschriebenen Herzpumpeneinrichtung. Das System kann weiterhin die für die extrakorporale Verwendung vorgesehene weitere Steuereinheit umfassen.

Der Schalter kann eingerichtet sein, infolge einer Störung und/oder eines Ausfalls der ersten Steuereinheit von dem Hauptbetriebszustand in den Hilfsbetriebszustand umzuschalten. Somit gewährleistet die vorgeschlagene Steuervorrichtung, dass die Herzpumpe auch bei der Störung beziehungsweise bei dem Ausfall der ersten Steuereinheit durch die zweite Steuereinheit weiterbetrieben wird. Ein Patient, bei dem die Herzpumpeneinrichtung verwendet wird, erhält somit ausreichend Zeit, im Falle eines Ausfalls der ersten Steuereinheit medizinische Unterstützung zu suchen. Nachfolgend kann ein chirurgischer Eingriff zum Austausch der Steuervorrichtung oder zumindest der ersten Steuereinheit durchgeführt werden.

Zur Erkennung der Störung und/oder des Ausfalls der ersten Steuereinheit kann es vorgesehen sein, dass beispielsweise kontinuierlich oder periodisch ein keep-alive-Signal an eine Schaltersteuerung des Schalters geschickt wird. Fällt dieses keep-alive-Signal aus, kann es vorgesehen sein, dass der Schalter vom Hauptbetriebszustand in den Hilfsbetriebszustand umschaltet. Zusätzlich oder alternativ könnte die erste Steuereinheit oder eine andere Überwachungseinheit der Steuervorrichtung beispielsweise permanent einen Strom der ersten Steuereinheit überwachen. Übersteigt oder unterschreitet dieser Strom einen festgelegten Grenzwert, schaltet der Schalter von dem Hauptbetriebszustand in den Hilfsbetriebszustand. Es kann zusätzlich vorgesehen sein, dass die erste Steuereinheit im Hilfsbetriebszustand abschaltet. Zusätzlich oder alternativ kann es auch vorgesehen sein, dass die erste Steuereinheit oder die Überwachungseinheit eine Temperatur der ersten Steuereinheit überwacht. Übersteigt die Temperatur der ersten Steuereinheit einen festgelegten Grenzwert, kann der Schalter von dem Hauptbetriebszustand in den Hilfsbetriebszustand umschalten.

Indem sowohl die erste als auch die zweite Steuereinheit implantierbar ausgeführt sind und die Steuervorrichtung die Schnittstelle zur transkutanen Übertragung aufweist, kann die Herzpumpeneinrichtung betrieben werden, ohne dass eine Hautdurchtrittsstelle für eine perkutane Verbindungsleitung zur Energieversorgung und Steuerung der Pumpe erforderlich ist. Dadurch kann ein Infektionsrisiko des Patienten gesenkt werden.

Im Hauptbetriebszustand ist die zweite Steuereinheit typischerweise nicht elektrisch mit der Herzpumpe verbunden. Es kann vorgesehen sein, dass die zweite Steuereinheit im Hauptbetriebszustand, insbesondere galvanisch und/oder spannungsfrei, von der Herzpumpe getrennt ist. Außerdem ist im Hilfsbetriebszustand typischerweise die erste Steuereinheit nicht elektrisch mit der Herzpumpe verbunden. Es kann vorgesehen sein, dass die erste Steuereinheit im Hilfsbetriebszustand, insbesondere galvanisch und/oder spannungsfrei, von der Herzpumpe getrennt ist. Entsprechende Trennungen der elektrischen Verbindungen können durch den Schalter vorgenommen werden. Der Schalter kann beispielsweise ein Relais oder einen Transistor als Schalteinheit umfassen. Typischerweise weist der Schalter eine Schaltersteuerung auf, die mit der Schalteinheit verbunden ist und die die Schalteinheit steuert. Die Schaltersteuerung ist typischerweise als Logik-Schaltkreis ausgeführt. Die Schaltersteuerung kann zum Erfassen einer Störung oder eines Ausfalls der ersten Steuereinheit mit dieser verbunden sein. Die Schaltersteuerung ist typischerweise eingerichtet, infolge der Störung oder des Ausfalls ein Schalten des Schalters auszulösen, um ein Umschalten von dem Hauptbetriebszustand in den Hilfsbetriebszustand zu bewirken.

Es kann vorgesehen sein, dass die erste Steuereinheit und die zweite Steuereinheit in einem einzigen Gehäuse mit einer äußeren Oberfläche aus biokompatiblem Material angeordnet sind, damit für die zweite Steuereinheit keine weitere implantierbare Komponente benötigt wird. Es kann aber auch vorgesehen sein, dass die erste Steuereinheit und die zweite Steuereinheit in jeweiligen Gehäusen mit äußeren Oberflächen aus biokompatiblem Material angeordnet sind. Die Gehäuse können eine biokompatible Hülle und/oder eine biokompatible Beschichtung aufweisen.

Die erste Steuereinheit kann eingerichtet sein, ein Schaltsignal an den Schalter zu senden. Außerdem kann der Schalter eingerichtet sein, bei einem Fehlen des Schaltsignals in den Hilfsbetriebszustand zu schalten, beziehungsweise den Hilfsbetriebszustand beizubehalten. Dadurch wird gewährleistet, dass der Schalter bei einem Ausfall der ersten Steuereinheit zuverlässig in den Hilfsbetriebszustand umschaltet, falls hierbei das Fehlen des Schaltsignals auftritt.

Die Steuervorrichtung kann eine implantierbare Hauptstromversorgung und eine implantierbare Hilfsstromversorgung aufweisen. Hierbei kann die Hauptstromversorgung eingerichtet sein, die erste Steuereinheit mit Energie zu versorgen. Außerdem kann die Hilfsstromversorgung eingerichtet sein, die zweite Steuereinheit mit Energie zu versorgen. Dadurch wird gewährleistet, dass auch im Falle eines Ausfalls der Hilfsstromversorgung oder der Hauptstromversorgung entweder die zweite oder die erste Steuereinheit mit elektrischer Energie versorgt wird und dass die Herzpumpe funktionsfähig bleibt. Es kann vorgesehen sein, dass die Hauptstromversorgung und die Hilfsstromversorgung räumlich voneinander getrennt, insbesondere in unterschiedlichen implantierbaren Gehäusen, angeordnet sind. Beispielsweise kann die Hauptstromversorgung oder die Hilfsstromversorgung derart angeordnet sein, dass diese zum Austausch, beispielsweise nachdem eine Anzahl maximaler Ladezyklen erreicht wurde, chirurgisch besonders leicht zu erreichen ist.

Es kann zusätzlich oder alternativ eine implantierbare Stromversorgung vorgesehen sein, die eingerichtet ist, sowohl die erste Steuereinheit als auch die zweite Steuereinheit mit Energie zu versorgen. Dadurch, dass die erste und die zweite Steuereinheit durch dieselbe Stromversorgung mit elektrischer Energie versorgt werden, kann ein kompakter Aufbau der Steuervorrichtung erreicht werden.

Eine elektrische Verbindung zwischen der Stromversorgung und der ersten Steuereinheit kann im Hilfsbetriebszustand, beispielsweise galvanisch und/oder spannungsfrei, trennbar sein. Auf diese Weise kann verhindert werden, dass die erste Steuereinheit die Stromversorgung im Hilfsbetriebszustand belastet, so dass eine Langlebigkeit der Steuervorrichtung im Hilfsbetriebszustand gewährleistet wird. Die elektrische Verbindung zwischen der Stromversorgung und der ersten Steuereinheit kann hierbei infolge eines Umschaltens oder bei einem Umschalten von dem Hauptbetriebszustand in den Hilfsbetriebszustand getrennt werden.

Die Schnittstelle weist typischerweise eine Sende- und/oder Empfangsspule auf. In einigen Ausführungen kann die Schnittstelle zusätzlich oder alternativ eine Funkantenne aufweisen. In typischen Ausführungen ist die Stromversorgung beziehungsweise die Hauptstromversorgung und/oder die Hilfsstromversorgung aufladbar. Typischerweise ist die Stromversorgung beziehungsweise die Hauptstromversorgung und/oder die Hilfsstromversorgung über die Schnittstelle drahtlos aufladbar. Somit kann ein Betrieb einer implantierten Herzpumpeneinrichtung über einen langen Zeitraum gewährleistet werden. In typischen Ausführungen sind die Stromversorgung, die Hauptstromversorgung und/oder die Hilfsstromversorgung als Batterien oder Akkumulatoren ausgebildet.

Der Schalter und/oder die erste Steuereinheit und/oder die zweite Steuereinheit können derart ausgeführt sein, dass ein Umschalten von dem Hauptbetriebszustand in den Hilfsbetriebszustand über eine drahtlose und transkutane Verbindung auslösbar ist. Beispielsweise kann ein Umschalten in den Hilfszustand durch Annähern eines Magneten an den Schalter und/oder an die erste Steuereinheit und/oder an die zweite Steuereinheit auslösbar sein. Zu diesem Zweck kann die Steuervorrichtung beispielsweise einen Reedschalter aufweisen. Somit kann der Hilfsbetriebszustand manuell durch einen Anwender ausgelöst werden, wenn beispielsweise eine automatische Erkennung eines Ausfalls oder einer Störung der ersten Steuereinheit fehlschlägt.

Es kann beispielsweise in einigen Ausführungen vorgesehen sein, dass die Steuervorrichtung keine mit der ersten Steuereinheit und/oder mit der zweiten Steuereinheit elektrisch verbundenen Schnittstellen für eine ausschließlich kabeigebundene Verbindung zwischen der weiteren Steuereinheit und der Steuervorrichtung aufweist.

Die vorliegende Anmeldung betrifft auch ein Verfahren zum Betrieb einer wie oben oder unten beschriebenen Herzpumpeneinrichtung oder einer wie oben oder unten beschriebenen Steuervorrichtung. Der Schalter schaltet hierbei infolge der Störung und/oder des Ausfalls der ersten Steuereinheit von dem Hauptbetriebszustand in den Hilfsbetriebszustand um.

In typischen Ausführungen wird die Herzpumpe im Hilfsbetriebszustand mit geringerer Leistung betrieben als im Hauptbetriebszustand. Es kann auch vorgesehen sein, dass die Sensoren im Hilfsbetriebszustand abgeschaltet oder mit geringerer Leistung betrieben werden. Beispielsweise kann es vorgesehen sein, dass die Herzpumpe im Hilfsbetriebszustand mit konstanter Rotordrehzahl betrieben wird. Zudem kann vorgesehen sein, dass die Betriebsparameter der Herzpumpe im Hauptbetriebszustand basierend auf einem Sensorsignal gesteuert werden. Es kann auch vorgesehen sein, dass die Betriebsparameter im Hilfsbetriebszustand unabhängig von diesem Sensorsignal gesteuert werden. Durch jede dieser Ausführungen ist ein besonders kompakter Aufbau der zweiten Steuereinheit möglich. Zudem kann erreicht werden, dass ein Energiebedarf der Steuervorrichtung im Hilfszustand reduziert wird. Somit kann erreicht werden, dass die Steuervorrichtung im Hilfszustand hinreichend langlebig ist, sodass der Patient rechtzeitig vor Ausfall der Herzpumpe medizinische Unterstützung bekommen kann.

Zur Erfassung des Sensorsignals kann die Herzpumpe oder die Herzpumpeneinrichtung einen Sensor oder mehrere Sensoren beziehungsweise die oben schon beschriebenen Sensoren aufweisen. Dieser Sensor ist beziehungsweise diese Sensoren sind typischerweise elektrisch mit der ersten Steuereinheit verbunden. Der Sensor beziehungsweise die Sensoren kann/können eine Auswahl folgender Sensortypen umfassen: Positionssensor für einen Rotor der Herzpumpe, Lage- und/oder Beschleunigungssensor, Volumenstromsensor, Temperatursensor, Drucksensor, Druckdifferenzsensor, Sauerstoffsättigungssensor, Flusssensor, chemischer Sensor zur Blutanalyse.

Eine kompakte Bauweise der Steuervorrichtung erlaubt ein hohes Maß an geometrischer Gestaltungsfreiheit. Beispielweise kann ein implantierbares Pumpengehäuse vorgesehen sein, indem die Herzpumpe und die zweite Steuereinheit aufgenommen sein können. Es kann auch vorgesehen sein, dass der Schalter in dem Pumpengehäuse aufgenommen ist.

In einigen Ausführungen können auch die erste Steuereinheit und der Schalter in demselben Gehäuse aufgenommen sein. Dadurch kann ein besonders kompakter Aufbau erreicht werden. Dies gilt insbesondere dann, wenn auch die zweite Steuereinheit in diesem Gehäuse aufgenommen ist. Typischerweise werden für die Herzpumpeneinrichtung lediglich zwei implantierbare Gehäuse benötigt, von denen die Herzpumpe, die erste Steuereinheit, die zweite Steuereinheit und der Schalter aufgenommen werden. Dadurch kann auch die Anzahl von notwendigen intrakorporalen Verbindungsleitungen reduziert werden.

In typischen Ausführungen sind ein die erste Steuereinheit und/oder die zweite Steuereinheit aufnehmendes Gehäuse und das Pumpengehäuse über eine subkutane Verbindung (Driveline) miteinander verbunden, die eine Länge von mindestens 15 cm und/oder höchstens 50 cm aufweist. Zur Anordnung der Herzpumpeneinrichtung in einem Patientenkörper ist diese subkutane Verbindung typischerweise biegsam.

Es kann außerdem vorgesehen sein, dass die Steuervorrichtung eingerichtet ist, ein Alarmsignal bei einem Ausfall der ersten und/oder der zweiten Steuereinheit abzugeben. In diesem Fall kann die entsprechende Komponente durch einen operativen Eingriff ausgetauscht werden.

Es kann außerdem eine extrakorporale Energieversorgung, beispielsweise eine Batterie, vorgesehen sein. Es kann vorgesehen sein, dass die Herzpumpe im Betrieb durch die extrakorporale Energieversorgung mit elektrischer Energie versorgt wird. Es kann vorgesehen sein, dass die Energie durch eine drahtlose transkutane Verbindung übertragen wird. Zu diesem Zweck kann die Steuervorrichtung beispielsweise eine Energieempfangsspule aufweisen. Zusätzlich kann auch die Schnittstelle zum Energieempfang von der extrakorporalen Energieversorgung eingerichtet sein. Es kann in einigen Ausführungen aber auch vorgesehen sein, dass die Herzpumpe, zumindest für kurze Zeitintervalle, durch die implantierbare Stromversorgung und/oder die Hauptstromversorgung und/oder die Hilfsstromversorgung mit elektrischer Energie versorgt wird. Dies kann insbesondere dann vorgesehen sein, wenn eine mit der extrakorporalen Energieversorgung verbundene und induktiv mit der Energieempfangsspule gekoppelte extrakorporale Energiesendespule für das Zeitintervall vorübergehend entfernt wird, beispielsweise während einer Körperpflege des Patienten.

Ausführungsbeispiele werden nachfolgend anhand der Abbildungen beschrieben. Es zeigen
- Fig. 1: eine schematische Ansicht eines Systems mit einer Herzpumpeneinrichtung und einer weiteren Steuereinheit gemäß einer ersten Ausführung,
- Fig. 2: eine schematische Ansicht eines Systems gemäß einer zweiten Ausführung,
- Fig. 3: eine schematische Ansicht eines Systems gemäß einer dritten Ausführung und
- Fig. 4: eine schematische Ansicht eines Systems gemäß einer vierten Ausführung.

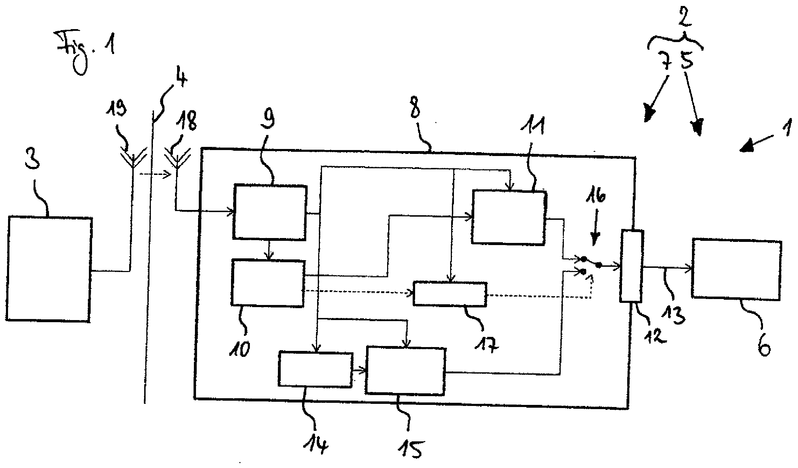

In Fig. 1 ist ein System 1 dargestellt, das eine implantierte Herzpumpeneinrichtung 2 und eine extrakorporale Steuereinheit 3 umfasst. Eine Hautoberfläche ist schematisch durch eine mit dem Bezugszeichen 4 versehene Linie gezeigt, wobei ein Körperinneres als ein von der Hautoberfläche 4 rechts- und ein Äußeres als ein von der Hautoberfläche 4 linksliegender Bereich zu verstehen ist.

Die Herzpumpeneinrichtung 2 weist eine Herzpumpe 5 auf. Die Herzpumpe 5 weist zumindest einen in einem implantierbaren, biokompatiblen und fluiddicht verschweißten Pumpengehäuse 6 angeordneten Motor und einen von dem Motor angetriebenen Rotor auf und kann derart angeordnet sein, dass durch eine Drehung des Rotors Blut vom linken Herzventrikel in die Aorta förderbar ist (sog. LVAD).

Außerdem umfasst die Herzpumpeneinrichtung 2 eine Steuervorrichtung 7. Die Steuervorrichtung 7 ist implantierbar und weist beispielsweise ein fluiddicht verschweißtes und biokompatibles Gehäuse 8 auf. Über eine Pumpenschnittstelle 12 und eine intrakorporale Driveline 13 ist die Steuervorrichtung 7 mit der Herzpumpe 5 verbunden. In dem dargestellten Beispiel ist in dem Gehäuse 8 ist eine Stromversorgung 9 in Form eines Akkumulators angeordnet. Zudem ist in dem Gehäuse 8 eine erste Steuereinheit mit einem ersten Mikrocontroller 10 und einer mit dem ersten Mikrocontroller 10 verbundenen ersten Motorsteuerung 11 angeordnet, wobei diese Komponenten einen Hauptpfad zur Steuerung und ggf. zum Antrieb der Herzpumpe 5 bilden. Die erste Motorsteuerung 11 umfasst typischerweise zumindest einen Mikroprozessor.

Zudem ist in dem Gehäuse 8 eine zweite Steuereinheit mit einem zweiten Mikrocontroller 14 und einer mit dem zweiten Mikrocontroller 14 verbundenen zweiten Motorsteuerung 15 angeordnet, wobei diese Komponenten einen Hilfspfad zur Steuerung und ggf. zum Antrieb der Herzpumpe 5 bilden. Es kann in einigen Ausführungen auch vorgesehen sein, dass die erste Steuereinheit und die zweite Steuereinheit in jeweiligen implantierbaren Gehäusen angeordnet sind. Die zweite Motorsteuerung 15 umfasst typischerweise zumindest einen Mikroprozessor. Die Steuereinheiten sind eingerichtet, Betriebsparameter der Herzpumpe 5, wie beispielsweise eine Rotordrehzahl und/oder einen Motorstrom und/oder eine Antriebsdrehzahl und/oder Signale zur Ansteuerung von implantierten Sensoren, zu steuern. Zu diesem Zweck können beispielsweise die Mikrocontroller 10, 14 gespeicherte Kennfelddaten umfassen, anhand derer die Betriebsparameter abhängig von Eingangsparametern, beispielsweise weiter unten beschriebenen Sensordaten, steuerbar sind. Die Mikrocontroller 10, 14 stellen den jeweiligen Motorsteuerungen 11, 15 beispielsweise über eine digitale Schnittstelle ein Steuersignal, zum Beispiel eine Soil-Motordrehzahl und insbesondere im Hilfsbetriebszustand direkt ein PWM- oder Sinus-Schaltsignal, zur Verfügung. Die Motorsteuerungen 11, 15 stellen auf Basis des Steuersignals den Strom für die Motorphasen zur Verfügung. Hierbei werden zyklisch immer zwei Phasen bestromt und von einer Phase wird das Back-EMF ausgewertet.

In dem Gehäuse 8 ist außerdem ein Schalter mit einer Schalteinheit 16 angeordnet, die mit dem Hauptpfad, dem Hilfspfad und über die Driveline 13 mit der Herzpumpe 5 verbunden ist. Die Schalteinheit 16 ist eingerichtet, zwischen einem Hauptbetriebszustand, in dem der Hauptpfad zum Steuern der Herzpumpe verwendet wird, und einem Hilfsbetriebszustand, in dem der Hilfspfad zum Steuern der Herzpumpe verwendet wird, umzuschalten. Zu diesem Zweck ist die Schalteinheit 16, beispielsweise ein Transistorschalter, eingerichtet, eine entsprechende elektrische Verbindung zwischen der Herzpumpe 5 und dem Hauptpfad oder dem Hilfspfad herzustellen beziehungsweise zu trennen.

Mit der Schalteinheit 16 ist eine Schaltersteuerung 17 verbunden, wobei die Schalteinheit 16 und die Schaltersteuerung 17 sowie Verbindungsleitungen zwischen diesen Komponenten den Schalter bilden. Die Schaltersteuerung 17 ist als Logikschaltkreis ausgeführt und mit dem ersten Mikrocontroller 10 verbunden. Wenn die Schaltersteuerung 17 eine Störung oder einen Ausfall des ersten Mikrocontrollers 10 und/oder der ersten Motorsteuerung 11 detektiert, löst die Schaltersteuerung 17 ein Umschalten der Schaltereinheit 16 aus, so dass die Steuervorrichtung 7 vom Hauptbetriebszustand in den Hilfsbetriebszustand umschaltet. Es kann hierbei beispielsweise vorgesehen sein, dass die Schaltersteuerung 17 permanent oder periodisch ein elektrisches Signal von dem ersten Mikrocontroller 10 erhält, wenn der erste Mikrocontroller 10 und/oder die erste Motorsteuerung 11 ordnungsgemäß funktioniert. Die Schaltersteuerung 17 kann eingerichtet sein, ein Umschalten auszulösen, wenn dieses Signal ausbleibt.

Die Steuervorrichtung 7 weist außerdem eine Schnittstelle 18 für eine drahtlose, transkutane Kommunikation mit der extrakorporalen Steuereinheit 3 auf. Die Herzpumpeneinrichtung 2 weist allerdings keinerlei Schnittsteflen für eine rein kabelgebundene Verbindung mit der extrakorporalen Steuereinheit 3 oder anderen extrakorporalen Komponenten auf. Die Schnittstelle 18 umfasst typischerweise eine Sende- und Empfangsspule zum induktiven Senden und Empfangen von Daten und zum Empfangen von Energie. Zusätzlich oder alternativ kann die Schnittstelle 18 beispielsweise einen Transceiver mit einer Sende- oder Empfangsantenne für eine funkgebundene Datenübertragung, beispielsweise im HF-Bereich, zwischen der Steuervorrichtung 7 und der extrakorporalen Steuereinheit 3 aufweisen. Über die Schnittstelle 18 kann beispielsweise die erste und/oder die zweite Steuereinheit und/oder die Schaltersteuerung 17 programmiert werden und/oder die Stromversorgung 9 geladen werden. Die extrakorporale Steuereinheit 3 umfasst eine entsprechend geeignete Schnittstelle 19.

Die Steuervorrichtung 7 ist zudem eingerichtet, durch ein Auflegen eines hinreichend starken Magneten auf eine Außenseite der Hautoberfläche 4 im Bereich der Schnittstelle 18 in den Hilfsbetriebszustand umzuschalten. Zu diesem Zweck weist beispielsweise die erste Steuereinheit und/oder die zweite Steuereinheit und/oder die Schaltersteuerung 17 einen Reedschalter und einen entsprechenden Schaltkreis auf. Der Reedschalter ist zusätzlich zum Schalter 16 vorgesehen.

Die zweite Steuereinheit mit dem zweiten Mikrocontroller 14 und der zweiten Motorsteuerung 15 ist deutlich kompakter ausgeführt als die erste Steuereinheit. Wird die Herzpumpeneinrichtung 2 im Hilfsbetriebsmodus betrieben, so ist die durch die zweite Steuereinheit festgelegte Rotordrehzahl der Herzpumpe 5 konstant und im Vergleich zum Hauptbetriebsmodus reduziert, so-dass die Herzpumpe 5 im Hilfsbetriebsmodus mit einer reduzierten Leistung betrieben wird. Zudem werden die Betriebsparameter der Herzpumpe 5 im Hauptbetriebszustand durch die erste Steuereinheit auch anhand von Sensorsignalen festgelegt. Die Sensorsignale, die beispielsweise eine Position des Rotors der Herzpumpe 5 und/oder eine Vielzahl weiterer Werte enthalten können, werden hierbei von den unterschiedlichen in der Figur nicht dargestellten implantierten Sensoren erfasst und an die erste Steuereinheit übertragen. Die von der zweiten Steuereinheit im Hilfsbetriebsmodus gesteuerten Betriebsparameter sind hingegen unabhängig von diesen Sensordaten.

Im Hauptbetriebszustand kann die Drehzahl der Herzpumpe beispielsweise durch den Mikrocontroller 10 anhand von Kennfelddaten und/oder den Sensordaten eingestellt werden. Hierbei kann eine Regelung der Drehzahl auch dynamisch erfolgen, zum Beispiel für einen Ansaugschutz, und/oder periodisch variierend für eine Erzeugung eines künstlichen Pulses.

Im Hilfsbetriebszustand stellt der Mikrokontroller 14 typischerweise eine konstante Drehzahl ein. Hierfür kann beispielsweise eine Messung des Motorstroms verwendet werden. Hierbei können also gewisse, im Hauptbetriebszustand vorgesehene Funktionen, im Hilfsbetriebszustand abgeschaltet oder nicht vorhanden sein, wie beispielsweise der Ansaugschutz und/oder eine periodische Änderung der Drehzahl und/oder die Auswertung des Back-EMF-Signals, so dass im Hilfsbetriebszustand beispielsweise eine Lage des Rotors nicht ausgewertet und/oder berücksichtigt wird.

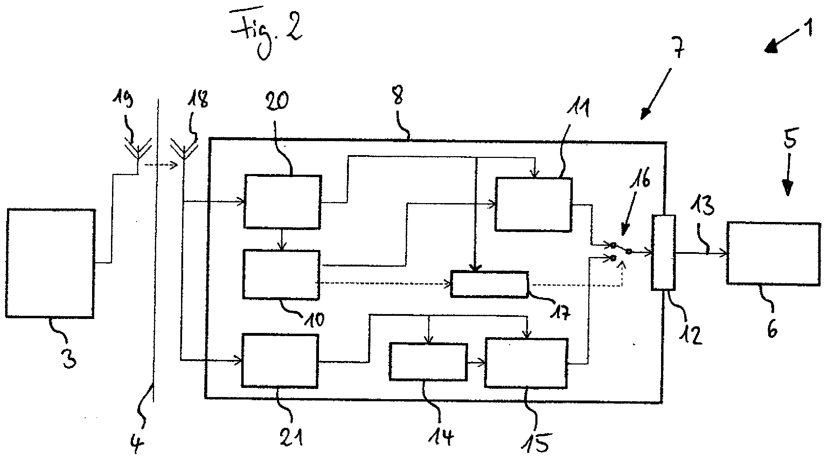

Die Stromversorgung 9 versorgt in dem in Fig. 1 dargestellten Beispiel die Mikrocontroller 10, 14, die Motorsteuerungen 11, 15 und die Schaltersteuerung 17 mit elektrischer Energie. Die Energie zum Antrieb der Herzpumpe 5 liefert eine nicht dargestellte extrakorporale Batterie, die mit der Steuervorrichtung, insbesondere mit der Schnittstelle 18 induktiv verbunden ist. Das in Fig. 2 dargestellte Ausführungsbeispiel des Systems 1 unterscheidet sich dahingehend, dass die Steuervorrichtung 7 eine in dem Gehäuse 8 angeordnete und mit dem ersten Mikrocontroller 10 und der ersten Motorsteuerung 11 verbundene Hauptstromversorgung 20 und eine ebenfalls in dem Gehäuse 8 angeordnete und mit dem zweiten Mikrocontroller 14 und der zweiten Motorsteuerung 15 verbundene Hilfsstromversorgung 21 aufweist. Wiederkehrende Merkmale sind in dieser und in den nachfolgenden Abbildungen mit den gleichen Bezugszeichen versehen. Sowohl die Hauptstromversorgung 20 als auch die Hilfsstromversorgung 21 sind Akkumulatoren und über die Schnittstelle 18 aufladbar. Die Schaltersteuerung 17 wird in dem dargestellten Beispiel wie der erste Mikrocontroller 10 und die erste Motorsteuerung 11 durch die Hauptstromversorgung 20 mit Energie versorgt.

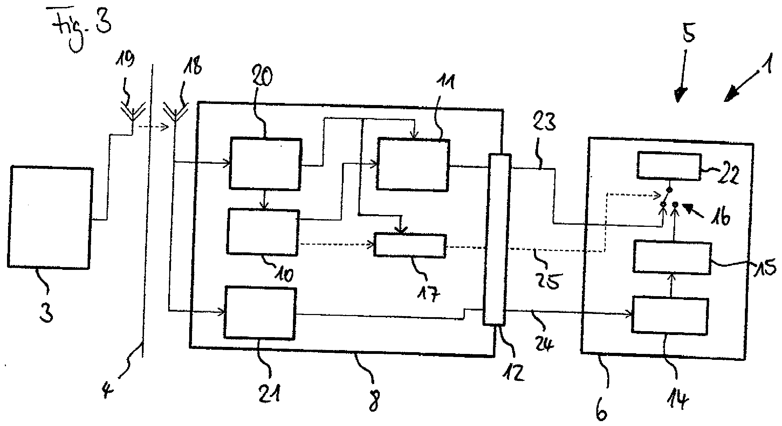

Ein weiteres Ausführungsbeispiel des Systems 1 ist in Fig. 3 gezeigt. Dieses System 1 unterscheidet sich von dem mit Bezug auf Fig. 2 beschriebenen System dadurch, dass sowohl die Schalteinheit 16 als auch die zweite Steuereinheit in dem Pumpengehäuse 6 angeordnet sind. Zusätzlich ist in Fig. 3 der mit der Schalteinheit 16 elektrisch verbundene Motor 22 der Herzpumpe 5 gezeigt. Die Pumpenschnittstelle 12 umfasst in diesem Fall drei Durchgänge, einen ersten Durchgang für eine Driveline 23 zur Übertragung von Betriebsparametern von der ersten Steuereinheit zu der Schalteinheit 16, einen zweiten Durchgang für eine Verbindungsleitung 24 zwischen der Hilfsstromversorgung und der zweiten Steuereinheit sowie einen dritten Durchgang für eine Verbindungsleitung 25 zwischen der Schaltersteuerung 17 und der Schalteinheit 16.

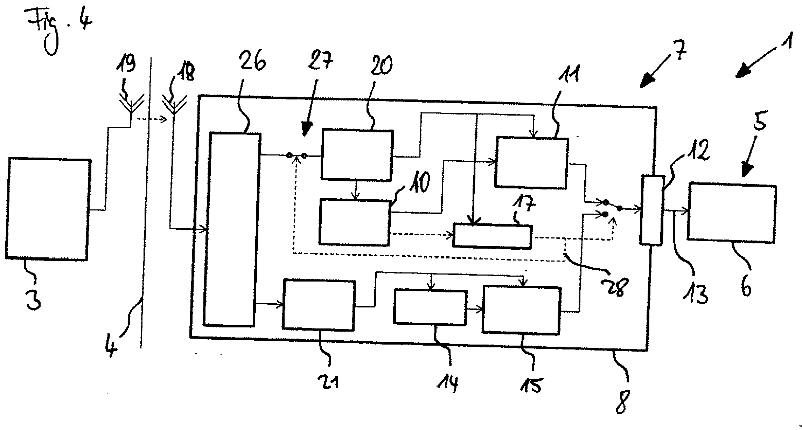

Ein weiteres Ausführungsbeispiel des Systems 1 ist in Fig. 4 gezeigt. Dieses System 1 unterscheidet sich von dem in Fig. 2 gezeigten System dadurch, dass ein weiterer Akkumulator 26 vorgesehen ist, der über die Schnittstelle 18 induktiv aufladbar ist und der die Hauptstromversorgung 20 und die Hilfsstromversorgung 21 mit Energie versorgt. Außerdem ist die Verbindung zwischen dem Akkumulator 26 und der Hauptstromversorgung 20 über einen weiteren Schalter 27 galvanisch und/oder spannungsfrei trennbar ausgeführt. Über eine schematisch gezeigte Verbindung 28 zwischen der Schaltersteuerung 17 und dem weiteren Schalter 27 ist der weitere Schalter 27 derart steuerbar, dass die Verbindung zwischen dem Akkumulator 26 und der Hauptstromversorgung 20 getrennt wird, wenn die Schaltvorrichtung 7 von dem Hauptbetriebszustand in den Hilfsbetriebszustand umschaltet.

Lediglich in den Ausführungsbeispielen offenbarte Merkmale der verschiedenen Ausführungsformen können miteinander kombiniert und einzeln beansprucht werden.

## Patentansprüche

1. Steuervorrichtung (7) für eine implantierbare Herzpumpe (5), umfassend eine implantierbare erste Steuereinheit, die in einem Hauptbetriebszustand zum Steuern von Betriebsparametern der Herzpumpe (5) elektrisch mit der Herzpumpe (5) verbunden ist, weiterhin umfassend eine mit der ersten Steuereinheit elektrisch verbundene Schnittstelle (18) zur drahtlosen, transkutanen Datenübertragung und/oder zur drahtlosen, transkutanen Energieübertragung zwischen der ersten Steuereinheit und einer für eine extrakorporale Verwendung vorgesehenen weiteren Steuereinheit (3), wobei die Steuervorrichtung (7) weiterhin eine implantierbare zweite Steuereinheit, die in einem Hilfsbetriebszustand zum Steuern von Betriebsparametern der Herzpumpe (5) elektrisch mit der Herzpumpe (5) verbunden ist, und einen mit der ersten Steuereinheit und der zweiten Steuereinheit elektrisch verbundenen und implantierbaren Schalter (16, 17) umfasst, der eingerichtet ist, zwischen dem Hauptbetriebszustand und dem Hilfsbetriebszustand umzuschalten.

2. Steuervorrichtung (7) nach Anspruch 1, wobei die erste Steuereinheit und die zweite Steuereinheit in einem einzigen Gehäuse (8) mit einer äußeren Oberfläche aus biokompatiblem Material oder in jeweiligen Gehäusen mit äußeren Oberflächen aus biokompatiblem Material angeordnet sind.

3. Steuervorrichtung (7) nach einem der Ansprüche 1 oder 2, wobei der Schalter (16, 17) eingerichtet ist, infolge einer Störung und/oder eines Ausfalls der ersten Steuereinheit von dem Hauptbetriebszustand in den Hilfsbetriebszustand umzuschalten.

4. Steuervorrichtung (7) nach einem der Ansprüche 1 bis 3, wobei die erste Steuereinheit eingerichtet ist, ein Schaltsignal an den Schalter (16, 17) zu senden, wobei der Schalter (16, 17) eingerichtet ist, bei einem Fehlen des Schaltsignals in den Hilfsbetriebszustand zu schalten beziehungsweise den Hilfsbetriebszustand beizubehalten.

5. Steuervorrichtung (7) nach einem der Ansprüche 1 bis 4, wobei die erste Steuereinheit und der Schalter (16, 17) in demselben Gehäuse (8) aufgenommen sind.

6. Steuervorrichtung (7) nach einem der Ansprüche 1 bis 5 mit einer implantierbaren Hauptstromversorgung (20) und einer implantierbaren Hilfsstromversorgung (21), wobei die Hauptstromversorgung (20) eingerichtet ist, die erste Steuereinheit mit Energie zu versorgen, und wobei die Hilfsstromversorgung (21) eingerichtet ist, die zweite Steuereinheit mit Energie zu versorgen.

7. Steuervorrichtung (7) nach einem der Ansprüche 1 bis 6 mit einer implantierbaren Stromversorgung (26), die eingerichtet ist, sowohl die erste Steuereinheit als auch die zweite Steuereinheit mit Energie zu versorgen.

8. Steuervorrichtung (7) nach Anspruch 7, wobei eine elektrische Verbindung zwischen der Stromversorgung und der ersten Steuereinheit im Hilfsbetriebszustand trennbar, insbesondere galvanisch trennbar, ist.

9. Steuervorrichtung (7) nach einem der Ansprüche 6 bis 8, wobei die Stromversorgung beziehungsweise die Hauptstromversorgung und/oder die Hilfsstromversorgung über die Schnittstelle (18) drahtlos aufladbar ist.

10. Steuervorrichtung (7) nach einem der Ansprüche 1 bis 9, wobei der Schalter (16, 17) und/oder die erste Steuereinheit und/oder die zweite Steuereinheit derart ausgeführt ist, dass ein Umschalten von dem Hauptbetriebszustand in den Hilfsbetriebszustand über eine drahtlose und transkutane Verbindung, insbesondere durch Annähern eines Magneten, auslösbar ist.

11. Herzpumpeneinrichtung (2), umfassend eine Steuervorrichtung (7) nach einem der Ansprüche 1 bis 10 und eine mit dem Schalter (16, 17) elektrisch verbundene Herzpumpe (5).

12. Herzpumpeneinrichtung (2) nach Anspruch 11 mit einem implantierbaren Pumpengehäuse (6), wobei die Herzpumpe (5) und die zweite Steuereinheit in dem Pumpengehäuse (6) aufgenommen sind.

13. System (1), umfassend eine Steuervorrichtung (7) nach einem der Ansprüche 1 bis 10 oder eine Herzpumpeneinrichtung (2) nach einem der Ansprüche 11 oder 12 und weiterhin umfassend die für die extrakorporale Verwendung vorgesehene weitere Steuereinheit (3).

14. Verfahren zum Betrieb einer Herzpumpeneinrichtung (2) nach einem der Ansprüche 11 oder 12 oder eines Systems (1) nach Anspruch 13, wobei der Schalter (16, 17) infolge einer Störung und/oder eines Ausfalls der ersten Steuereinheit von dem Hauptbetriebszustand in den Hilfsbetriebszustand umschaltet.

15. Verfahren nach Anspruch 14, wobei die Herzpumpe (5) im Hilfsbetriebszustand mit geringerer Leistung betrieben wird als im Hauptbetriebszustand.

16. Verfahren nach einem der Ansprüche 14 oder 15, wobei die Herzpumpe (5) im Hilfsbetriebszustand mit konstanter Rotordrehzahl betrieben wird.

17. Verfahren nach einem der Ansprüche 14 bis 16, wobei die Betriebsparameter der Herzpumpe (5) im Hauptbetriebszustand basierend auf einem Sensorsignal und im Hilfsbetriebszustand unabhängig von diesem Sensorsignal gesteuert werden.
